# EUROPEAN PATENT SPECIFICATION

(11) **EP 3 430 396 B1**
(45) Date of publication and mention of the grant of the patent: **06.01.2021**
(21) Application number: 17710889.1
(22) Date of filing: 14.03.2017
(51) Int. Cl.: G01N 33/50

(54) **3D-TISSUE CULTURE BASED METHOD TO ASSESS MITOCHONDRIAL IMPAIRMENT**
AUF 3D-GEWEBEKULTUR BASIERENDES VERFAHREN ZUR BEURTEILUNG DER MITOCHONDRIALEN BEEINTRÄCHTIGUNG
PROCÉDÉ BASÉ SUR UNE CULTURE DE TISSU 3D POUR ÉVALUER UNE DÉFICIENCE MITOCHONDRIALE

(30) Priority: 14.03.2016 GB 201604292
(43) Date of publication of application: 23.01.2019
(73) Proprietor: Insphero AG, 8952 Schlieren (CH)
(72) Inventor: KELM, Jens, 8952 Schlieren (CH); MESSNER, Simon, 8952 Schlieren (CH)
(74) Representative: Michalski Hüttermann & Partner Patentanwälte mbB
(86) International application number: PCT/EP2017/055999
(87) International publication number: WO 2017/157942

(56) References cited:
- US-A1- 2011 136 163
- SIMON MESSNER ET AL: "Mitotoxicity Testing in 3D Liver Microtissues : Novel Two-Step Assay Integrates Toxicity Testing in 3D Liver Models and Assessment of OCR", GENETIC ENGINEERING & BIOTECHNOLOGY NEWS, vol. 36, no. 16, 15 September 2016 (2016-09-15), pages 24-25, XP055373139, US ISSN: 1935-472X, DOI: 10.1089/gen.36.16.12
- ROESSGER K ET AL: "Assessment of mitochondrial activity in 3D human liver microtissues as a tool for mechanistic investigations of adverse drug effects", TOXICOLOGY LETTERS, vol. 258, 16 September 2016 (2016-09-16), XP029714581, ISSN: 0378-4274, DOI: 10.1016/J.TOXLET.2016.06.1856
- MESSNER S ET AL: "In-depth characterization of 3D human liver microtissues by transcriptomic, proteomic and functional analysis", TOXICOLOGY LETTERS, vol. 238, no. 2, 16 October 2015 (2015-10-16), XP029284904, ISSN: 0378-4274, DOI: 10.1016/J.TOXLET.2015.08.534
- MESSNER SIMON ET AL: "Organotypic 3Din vitromicrotissue models for cardiac and liver safety assessment", JOURNAL OF PHARMACOLOGICAL AND TOXICOLOGICAL METHODS, vol. 70, no. 3, 1 November 2014 (2014-11-01), page 328, XP029109658, ISSN: 1056-8719, DOI: 10.1016/J.VASCN.2014.03.068
- MESSNER S ET AL: "Multi-cell type human liver microtissues for hepatotoxicity testing", ARCHIVES OF TOXICOLOGY, SPRINGER-VERLAG, BERLIN, DE, vol. 87, no. 1, 11 November 2012 (2012-11-11), pages 209-213, XP035157583, ISSN: 1432-0738, DOI: 10.1007/S00204-012-0968-2
- STEFAN LETZSCH ET AL: "Quantifying Efflux Activity in 3D Liver Spheroids : New Confocal Imaging Instruments Allow Screening in Complex Human Liver Microtissues", GENETIC ENGINEERING & BIOTECHNOLOGY NEWS, vol. 35, no. 7, 1 April 2015 (2015-04-01), pages 14-15, XP055373280, US ISSN: 1935-472X, DOI: 10.1089/gen.35.07.08
- ALI KERMANIZADEH ET AL: "Hepatic toxicology following single and multiple exposure of engineered nanomaterials utilising a novel primary human 3D liver microtissue model", PARTICLE AND FIBRE TOXICOLOGY, BIOMED CENTRAL, LONDON, GB, vol. 11, no. 1, 20 October 2014 (2014-10-20), page 56, XP021201583, ISSN: 1743-8977, DOI: 10.1186/S12989-014-0056-2
- ROESSGER K ET AL: "Characterization and application of 3D Multi-Donor Human Liver Microtissues for predictive DILI testing and mechanistic investigations", TOXICOLOGY LETTERS, vol. 258, 16 September 2016 (2016-09-16), XP029714306, ISSN: 0378-4274, DOI: 10.1016/J.TOXLET.2016.06.1578
- ROESSGER K ET AL: "Evaluation of species-specific drug-induced liver injury in 3D rat, dog, monkey and human liver microtissues", TOXICOLOGY LETTERS, vol. 258, 16 September 2016 (2016-09-16), XP029714002, ISSN: 0378-4274, DOI: 10.1016/J.TOXLET.2016.06.1319
- NATALIE ALÉPÉE: "State-of-the-art of 3D cultures (organs-on-a-chip) in safety testing and pathophysiology", ALTERNATIVES TO ANIMAL EXPERIMENTATION : ALTEX, 1 January 2014 (2014-01-01), XP055324767, DE ISSN: 1868-596X, DOI: 10.14573/altex1406111

## Description

### Field of the invention

The present invention relates to a method and/or assay for the assessment of the metabolic effect of a candidate compound. The method and/or assay comprises exposing one or more 3-dimensional cell culture or tissue to a candidate compound.

Mitochondria play a critical role in generating most of the cell's energy as ATP. They are also involved in other metabolic processes such as urea generation, haem synthesis and fatty acid beta-oxidation. Disruption of mitochondrial function by drugs can result in cell death by necrosis or can signal cell death by apoptosis (e.g., following cytochrome c release).

Drugs that injure mitochondria usually do so by inhibiting respiratory complexes of the electron chain; inhibiting or uncoupling oxidative phosphorylation; inducing mitochondrial oxidative stress; or inhibiting DNA replication, transcription or translation.

It is important to test for mitochondrial toxicity early in drug development as impairment of mitochondrial function can induce various pathological conditions that are life threatening or can increase the progression of existing mitochondrial diseases.

Mitochondrial impairment has been reported to be involved, causative or at least related to different types of diseases, including Parkinson's disease, Alzheimer's disease, cardiac diseases, cancer, as well as cholesterol and lipid disorders.

Mitochondrial toxicity assays have been developed for this reason. Some of them, like the Mitochondrial ToxGlo™ Assay provided by Promega, comprise a cell-based assay method that uses a suspension of individual cells. The assay is based on the differential measurement of biomarkers associated with changes in cell membrane integrity and cellular ATP levels relative to vehicle-treated control cells during short exposure periods. Cell membrane integrity is first assessed by measuring the presence or absence of a distinct protease activity associated with necrosis. Next, ATP is measured after cell lysis. The two sets of data can be combined to produce profiles representative of mitochondrial dysfunction or non-mitochondrial related cytotoxic mechanisms.

Other Mitochondrial toxicity assays use 2d cell cultures, where cells are for example seeded in collagen I-coated microplates and adhered for 18-24 h before compound additions. For primary screening and secondary dose-response experiments, compounds at specific concentrations added to plated cells, and incubated for 24 h at 37°C, 5% CO₂. Then, cell markers are added to carry out imaging cytometry analyses, which result in a measure of mitochondrial toxicity (Tsiper et al, 2012). US 2011/136163 discloses a method for assessing toxicity of candidate agent to a sample of cells, involves providing a sample of cells, exposing the cells to the candidate agent for a suitable period of time, assaying the cells to measure data for at least one parameter of cellular function, and correlating the measured data of at least one parameter of cellular function with toxicity, where the exposing step is carried out in the presence of a reagent that facilitates the transport of the candidate agent into the cell.

It is one object of the present invention to improve the methods from the prior art. It is another object of the present invention to increase the predictability of methods from the prior art. It is still another object of the present invention to further develop methods from the prior at in that they allow the investigation of the effects of long term exposure of cells to a candidate compound. It is still another object of the present invention to increase the sensitivity of the methods from the prior art in that they provide a more sensitive and precise prediction of mitochondrial toxicity of candidate compounds. It is still another object of the present invention to allow detection of mitochondrial impairment at toxin concentrations which do not yet affect cell viability, and cannot be detected in cell viability/cytotoxicity assays or mitochondrial toxicity assays according to the prior art.

### Embodiments of the invention

These and further objects are met with methods and means according to the independent claims of the present invention. The dependent claims are related to specific embodiments. The scope of the invention is defined in the appended claims.

### Summary of the invention

Before the invention is described in detail, it is to be understood that this invention is not limited to the particular component parts of the devices described or process steps of the methods described as such devices and methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. It is moreover to be understood that, in case parameter ranges are given which are delimited by numeric values, the ranges are deemed to include these limitation values.

According to one embodiment of the invention, a method and/or assay for the assessment of the metabolic effect of a candidate compound is provided, which comprises
a) exposing one or more 3-dimensional cell culture or tissue to one or more candidate compounds, and
b) measuring, in at least one 3-dimensional cell culture or tissue, the effect of such exposure on the 3-dimensional cell culture or tissue-specific respiration rate (MTSRR).

The terms "3-dimensional cell culture or tissue" and "3D microtissue" are used interchangeably herein. Surprisingly, the inventors have shown that with this assay, mitochondrial impairment can be detected at toxin concentrations which do not yet affect cell viability, and cannot be detected in cell viability/cytotoxicity assays or mitochondrial toxicity assays according to the prior art.

The inventors attribute this finding to the fact that a 3-dimensional cell culture or tissue is a more faithful reproduction of a natural tissue. In other words: it behaves more physiologically than, e.g., a 2D cell culture or a cell suspension. The 3-dimensional cell culture or tissue recapitulates the smallest functional unit of a tissue or organ, providing a morphological and functional analog to native tissue. They thus bridge the gap between *in vitro* and *in vivo* toxicity testing models. Without being bound to theory, the inventors explain this phenomenon with different effects. First, in a 3-dimensional cell culture or tissue, an interstitium and an extracellular can be built up. Second, in a 3-dimensional cell culture or tissue, cells can develop 3-dimensonal cell-cell interactions. Third, in a 3-dimensional cell culture or tissue, drugs to be tested have to enter the tissue by diffusion from the outside, thus affecting cells in the outer cell mass more than cells in the inner cell mass. All these phenomena do not exist in 2D cell cultures and/or cell suspensions.

In one embodiment of the invention, the method and/or assay further comprises
c) measuring, in the same or at least one other 3-dimensional cell culture or tissue, the effect of such exposure on cell viability and/or the cytotoxic effect (CV-CYT) in one or more cells of at least one 3-dimensional cell culture or tissue, and
d) comparing the effects of steps b) and c) to provide an estimate on the mitochondrial toxicity of the one or more candidate compounds.

Steps a) and b), steps a) and c) and steps b) and c) can be carried out synchronously. In another embodiment, step a) can be carried out before steps b) and c), which can be carried out synchronously or subsequently.

As discussed already, mitochondrial toxicity assays from the prior art use either cell suspensions or 2D cell cultures. The inventors have unexpectedly shown that 3D microtissues exhibit a spare respiratory capacity which is more representative to the in vivo situation, i.e., to real tissues. Therefore, the use of 3D microtissues provides a more sensitive and precise prediction of mitochondrial toxicity of candidate compounds.

Generally, in drug discovery, preclinical testing, and clinical trials is estimated to cost between $500 million and $1.2 billion USD. Chances are that a drug will fail at any stage of development are enormous. Safety-related issues remain the primary reason for drug failure, largely because predicting which drugs will prove toxic in patients is extremely difficult. The standard assays discussed above suffer from limited throughput, loss of cell viability, and decrease in measurable tissue-specific functionality. *In vivo* animal tests are expensive, raise moral and ethical issues, and often fail to reveal important signs of toxicity in humans. In one further embodiment of the invention, said method and/or assay is thus used to determine the mitochondrial toxicity of a candidate compound. In this embodiment, the candidate compound is for example a candidate pharmaceutic, where potential toxic effects or other side effects are determined in a toxicity screening process. This embodiment also covers other compounds, like herbicides, fungicides or insecticides.

In one further embodiment of the invention, said method and/or assay is used to determine the chemotherapeutic efficacy of a candidate compound. In this embodiment, the candidate compound is a candidate chemotherapeutic, where the measured mitochondrial toxicity is a measure for potential chemotherapeutic efficacy.

In one further embodiment of the invention, the 3-dimensional cell culture or tissue is a spheroidal cell culture.

As discussed above, 3-dimensional cell culture or tissue have a significant advantage in the present context because they are a more faithful reproduction of a natural tissue, and behave more physiologically than, e.g., a 2D cell culture or a cell suspension. Further, they have a longer lifetime than 2D cell cultures or cell suspensions, which facilitates handling and allows for the testing of long term effects and off-target effects of candidate compounds. This is especially useful in screening of compounds that may later be used for long term treatments, or to which humans are exposed over long stretches of time.

In one further embodiment of the invention, said method and/or assay the 3-dimensional cell culture or tissue comprises primary cells. Compared to tumor cells or immortalized cells, primary cells have not accumulated mutations, and is thus more representative, or predictive, for the behavior of *in vivo* tissue. The transfer of primary cells into 3-dimensional cell cultures or tissues has two significant advantages: a) it further increases the physiologic similarity of the cells to *in vivo* tissues, and thus makes them even more representative, or predictive, and b) it increases the longevity of the primary cells compared to 2D cell cultures or cell suspensions.

Preferably, the 3-dimensional cell culture or tissue is produced by means of a hanging drop microtiter plate, as distributed by InspheroAG of Schlieren, Switzerland, under the brand "GravityPLUS™ Hanging Drop System", and disclosed in international patent application WO2010031194A1. This approach allows the generation of 3-dimensional cell cultures or tissues in more complex 3D cell culture scenarios, such as when using primary cells, cell lines that are resistant to self-aggregation, or when generating co-culture microtissues.

In another embodiment, the 3-dimensional cell culture or tissue is produced in a ultra-low attachment (ULA) microtiter plate, as distributed by InspheroAG of Schlieren, Switzerland, under the brand "GravityTRAP™ Ultra-Low Attachment (ULA) Plate".

In one further embodiment of the invention, said method and/or assay the 3-dimensional cell culture or tissue comprises hepatocytes. Hepatocytes in primary culture provide the closest *in vitro* model to human liver, which is the organ most effected by drug toxicity, and the only model that can produce a metabolic profile of a given drug that is similar to that found *in vivo.* Hepatocytes in primary culture express typical hepatic functions and express drug metabolizing enzymes. These advantages are particularly present in 3-dimensional cell cultures or tissues, which behave even more physiological, as the inventors have surprisingly shown, and do therefore more faithfully reflect the response of "true" liver tissue on drug exposure.

In other embodiments, the cells can be cardiac cells, neuronal cells, pancreatic islets, fibroblasts and keratinocytes. 3-dimensional cell cultures or tissues made from these cell types have specific applications in drug toxicity or efficacy screening.

In one further embodiment of the invention, the 3-dimensional cell culture or tissue comprises immortalized, malignant, cancerous and/or neoplastic cells.

In this embodiment, the effect of prior exposure on the microtissue-specific respiration rate (MTSRR) of the said cells can be used to investigate a potential chemotherapeutic efficacy of the tested molecule on cancer cells.

Further, cancer cells inherently alter their cellular machinery to change how they consume and utilize glucose. Inhibiting cancer specific key steps in the cellular respiration may disrupt cancer cell proliferation and survival without affecting normal cells.

In one further embodiment of the invention, the method and/or assay further comprises a step of determining, in the same or at least one other 3-dimensional cell culture or tissue, the effect of the exposure on the size thereof. This step can be carried out synchronously or subsequently with steps a) b) and/or c).

Preferably, the size determination refers to at least one parameter selected from the group consisting of:
- Diameter
- perimeter
- volume
- area of an optical cross section

The size can thus either be a parameter that has directly been measured, or a parameter which has been calculated on the basis of such measurements.

Preferably, the size determination of the 3-dimensional cell culture or tissue is carried out by means of an imaging device.

One example of such imaging device is the Cell³iMager distributed by, InSphero AG, Schlieren, CH, and manufactured by SCREEN, Japan. It allows analysis of spheroids by scanning multi-well plates in a bright-field. It computes estimated values based on spheroid size and density, together with spheroid number and area in each well. With easy and efficient operability, its vibration-free design protects cells from damage. An excellent application is also available to determine spheroid proliferation over time and to measure the granular distribution in 3D culture.

In another embodiment, the 3-dimensional cell culture or tissue further comprises at least one cell type selected from the group consisting of:
- Immune cells
- Stroma cells
- Fibroblasts
- Cancer stem cells
- Immortalized, malignant, cancerous and/or neoplastic cells that are known to be resistant or sensitive to a given anti-cancer agent,

Using immune cells in such 3-dimensional cell culture or tissue has a particular benefit because effects of the innate immune system can also be investigated. This is particularly useful in case an anti-cancer agent is investigated which co-acts with the immune system i.e., an immunotherapeutic agent, like an immune checkpoint inhibitor (see infra). These drugs activate the immune system to target cancer cells. Therefore, to test their efficacy *in vitro,* and to investigate potential relapses, these agents require the presence of immune cells.

Examples of suitable immune cells that can be used for such co-culture encompass, inter alia, tissue resident leukocytes, like macrophages (e.g., stellate macrophages/kupffer cells), CD8⁺ T cells, natural killer cells, natural killer T cells, and/or regulatory T cells.

Using connective tissue components, like stroma cells or fibroblasts, may help recreating the important tumor microenvironment in vitro, and thus increase the quality of the 3-dimensional cell culture or tissue, and this improve the predictive value of such culture or tissue for assays or screening purposes.

Cancer stem cells (CSCs) are cancer cells that possess characteristics associated with normal stem cells. Cancer stem cells sometimes share a feature with normal somatic stem cells in that they are naturally resistant to chemotherapeutic agents, because they because they express different transporter proteins, which actively export drugs. CSC are thus often able to survive first line cancer treatments and thus causing tumor relapse.

Co-culturing immortalized, malignant, cancerous and/or neoplastic cells with cancer stem cells can thus help to develop therapies which affect both the primary tumor and, at the same time, inhibit cancer stem cells.

The same rationale applies to the co-culture of a given immortalized, malignant, cancerous and/or neoplastic cell type with another immortalized, malignant, cancerous and/or neoplastic cell type that is known to be resistant or sensitive to a given anti-cancer agent.

In one embodiment, the microtissue-specific respiration rate is determined by simultaneous measurement of oxygen consumption rate (OCR) and extracellular acidification rate (ECAR), of one or more isolated 3D microtissues.

OCR is an indicator of mitochondrial respiration, and ECAR is largely the result of glycolysis. Such measurement can take place, e.g., in a Seahorse XF Analyzer, while other suitable devices exist on the market and can likewise be used. The Seahorse XF Analyzer is capable of performing real-time measurements of OCR and ECAR in extremely small volumes (about 2 µL) of medium above, usually, a monolayer of cells within a microplate well. Cellular oxygen consumption (respiration) and proton excretion (glycolysis) cause rapid, easily measurable changes to the concentrations of dissolved oxygen and free protons in this "transient microchamber" which are measured every few seconds by solid state sensor probes residing 200 microns above the cell monolayer. The instrument measures the concentrations for 2-5 minutes, and then calculates the OCR, and/or ECAR, respectively, e.g., in units of pmol/minute (OCR) and mpH/minute (ECAR). Total assay time is typically 60 to 90 minutes.

The inventors have for the first time shown that in this method, unexpectedly, 3D microtissues can be used instead of the cell monolayers that are disclosed in the secondary literature as well as in the product manual. Because of the microliter volumes that are provided by the wells, a spheroidal microtissue in such well provides a completely different microenvironment than a cell monolayer on the bottom thereof. Therefore, it was not foreseeable that a transfer of the respective method to 3D microtissues would be feasible and provide meaningful and reproducible results.

In one embodiment, in the determination of the microtissue-specific respiration rate (MTSRR), the spare respiratory capacity ("SPARE") is used.

Spare Respiratory Capacity indicates the capability of the cell to respond to an energetic demand as well as how closely the cell is to respiring to its theoretical maximum. Spare Respiratory Capacity is defined as the difference between maximal respiration and basal respiration of a tissue and/or cell.

In order to determine spare respiratory capacity, one approach is to first measure the basal respiration rate, and then measure the maximal respiration rate. The arithmetic difference ("delta") between the two is the spare respiratory capacity.

The basal respiration rate shows the energetic demand of the tissue or cell under baseline conditions, while the maximal oxygen consumption reflects the maximum rate of respiration a tissue or cell can achieve.

The basal respiration rate can be determined by measurement of oxygen consumption rate (OCR) in the first step of the measurement. The maximal respiration rate can be determined by measurement of oxygen consumption rate (OCR) in tissues or cells treated with an uncoupling agent, like carbonyl cyanide-4 (trifluoromethoxy) phenylhydrazone (FCCP). Such uncoupling agent mimic a physiological energy demand by stimulating the respiratory chain to operate at maximum capacity, by collapsing the proton gradient and disrupting the mitochondrial membrane potential. As a result, electron flow through the electron transport chain is uninhibited and oxygen is maximally consumed by complex IV.

According to further embodiments, in the determination of the microtissue-specific respiration rate (MTSRR), the basal respiration rate ("BASAL") and/or the maximal respiration rate ("MAX") are also used.

Primarily, the spare respiratory capacity (SPARE) is used in the determination of the microtissue-specific respiration rate (MTSRR), and basal respiration rate (BASAL) and maximal respiration rate (MAX) are used in case of inaccuracies or higher sensitivity compared to SPARE.

In one embodiment, the measurement of cell viability/cytotoxic effect determines the residual viability of one or more cells.

Cell viability/cytotoxic effect can for example be determined by quantitation of the ATP present in the cells. One such assay is the CellTiter-Glo Luminescent Cell Viability Assay (Promega, Madison, US), while other cell viability/cytotoxicity assays exist on the market and can likewise be used.

In another embodiment, the effect on microtissue-specific respiration rate and/or the effect on cell viability/cytotoxic effect is determined as inhibitory concentration, preferably as half maximal inhibitory concentration (IC₅₀).

Said inhibitory concentration refers to the concentration of the candidate compound, to which the 3-dimensional cell culture or tissue is exposed in step a), as compared to an untreated 3-dimensional cell culture or tissue.

The variable used herein for IC₅₀ with respect to microtissue-specific respiration rate is IC_{5O-MTSRR}. As discussed above, MTSRR is preferably determined by determining spare respiratory capacity (SPARE). In such case, IC_{5O_MTSRR} would be equal to IC_{5O_SPARE}.

The variable used herein for IC₅₀ with respect to cell viability/cytotoxicity is IC_{5O_CV/CYT}.

In one further embodiment exposure step a) is carried out prior to subsequent measurement steps b) and/or c).

This embodiment allows exposure times of different lengths. In such way, short term effects and long term effects can be investigated. This embodiment has a surprising synergism with the claimed use of 3-dimensional cell cultures or tissues, which, as discussed above, have better longevity as compared to 2D cell cultures or cell suspensions, and are thus suitable to long-term exposure to candidate compounds, like drug candidates.

In contrast thereto, the use instructions of the Seahorse XF Analyzers provide that the test drugs are dispensed to the cells during the assay. For this purpose, the device provides an assay cartridge that accommodates up to four drugs for automated injection during the assay. The assay cartridge is first placed into the analyzer to allow automatic calibration of optical sensors. Next, the cell culture plate is inserted into the instrument. In such setting, however, no long term exposure tests can be carried out.

In yet another embodiment, exposure step a) is carried out in one or more first vessels, while the subsequent measurement steps b) and/or c) are carried out in one or more second and/or third vessels.

Exposure is carried out, preferably, in vessels of a first array (e.g., wells of a first microtiter plate), while the subsequent measurement is carried out in vessels of a second array (e.g., wells of a 2^{nd} microtiter plate).

In still another embodiment, after exposure step a) the microtissues are transferred, by means of a suitable dispensing device, from the one or more first vessels to the one or more second and/or third vessels, to carry out measurement steps b) and/or c)

Said dispensing device is, e.g., a multichannel pipette with pipette tips that are either non-protein binding or pre-coated with a protein, to avoid that microtissues stick or adhere to the inner or outer surface of the pipette tip.

In another embodiment, the mitochondrial toxicity is determined on the basis of the quantitative relationship between
a) the IC₅₀ with respect to microtissue-specific respiration rate (IC_{5O_MTSRR})
b) the IC₅₀ with respect to cell viability/cytotoxicity (IC_{5O_CV/CYT}).

Preferably,
a) IC_{5O_MTSRR} ≥ IC_{5O_CV/CYT} means that the candidate compound has no specific mitochondrial toxicity
b) IC_{5O_MTSRR} < IC_{5O_CV/CYT} and IC_{5O_MTSRR} ≥ 0.75 x IC_{5O_CV/CYT} means that the candidate compound has a low risk of mitochondrial toxicity
c) IC_{5O_MTSRR} < 0.75 x IC_{5O_CV/CYT} means that the candidate compound has a high risk of mitochondrial toxicity

### Experiments and Figures

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

### Example 1: Generation of 3D microtissue

### 1.1. Thawing of cryopreserved Hepatocytes

- Take chosen cell-vial from cryo-tank and transfer into water bath (37 °C); set timer on 2 minutes
- Pipette 40 ml of the Wash/Thawing medium into 50 ml tube
- Transfer the hepatocytes into the tube, wash with 1 ml medium
- Place 50ml tube into centrifuge; start centrifugation for 5 min at 50 rcf (corresponds to 600rpm) at RT.
- Remove supernatant
- Wash pellet with 20ml wash buffer
- Place carefully 3 ml of Wash/Thawing medium into the 50ml tube
- Re-suspend cell pellet with 2D cell culture medium
- Count hepatocytes with e.g. Trypan Blue

### 1.2. Hepatocyte pre-plating

- Use a collagen coated cell-culture dish for pre-seeding
- Seed Hepatocytes in a 6 cm dish: 100000-250000 hepatocytes/cm²; 0.05-0.5ml per cm²
- Place at 37°C in a CO₂ containing incubator
- Optionally: Exchange medium after attachment with Serum-free 2D-culture medium
- Harvest hepatocytes after 12-96 hours (daily medium exchange if longer than 12hours)
- 3x wash with pre-warmed phosphate buffered saline the non-adherent hepatocytes away
- Add cell detachment solution such as collagenase/accutase mixtures to culture dish to dislodge the hepatocytes from the well 50-500 ul/cm²
- Incubate 5-30min at 37°C (visual observation until most cells are detached from the surface)
- Carefully transfer dispersed hepatocytes into a 50ml tube prefilled with wash bufffer
- Centrifuge for 5 min with 50 rcf (corresponds to 600 rpm) at RT
- Aspirate supernatant, add 1-50 ml wash medium to the pellet and re-suspend pellet by gentle pivoting of the tube
- Repeat centrifugation step for 5 min with 50 rcf (corresponds to 600 rpm) at RT
- Add 1 ml re-aggregation medium to the pellet and dissolve hepatocytes by gentle pivoting of the tube
- Count hepatocytes

### 1.3. Production of Microtissues

### A. Preparation of GravityPLUS™ plates (Insphero AG, CH)

- Fill Omnitray with 15 ml of 0.75x PBS/Amphothericin and add humidifier pad
- Place frame on Omnitray
- Put lid on the plate and label plate

### B. Preparation of cell suspension

- Prepare cell suspension of defined cell number (i.e. 25'000 Heps/25'000 NPCs per ml) in falcon tubes
- Homogenize cell suspension by pivoting the tube

### C. Seeding

- Use Viaflo electronic multichannel pipette (Integra Bioscience)
- Set Viaflo parameters: Repeat dispense: 40 ul, Speed 3, Repeat: 1x (for 96-well Viaflo)
- Empty cell suspension in reservoir (don't pour more than 30 ml in the reservoir, to ensure proper distribution of cells)
- Remove lid from GravityPLUS plates
- Prior Aspiration of cell suspension, gently pivot reservoir for homogeneous distribution of cells
- Start Viaflo program: Repeat dispense
- Place Viaflo horizontally on the inserts, avoid tapping of the drop on the top by gentle pressure of the Viaflo on the inserts
- Place lid back on plates

### D. Transfer of microtissues

- Pre-wet the GravityTRAP plate: Add 30-50µl medium or PBS to the very bottom of each well and remove by aspiration or alternatively by flicking out (use a soak matt)
- Place the GravityPLUS plate right next to the receiver bottom plate. Take both, lid and frame together off the reservoir, and carefully latch the pins of the frame (still with the lid on) into all 3 notches of the receiver plate
- Apply 70µl of medium which will release the hanging drop with the microtissue inside
- The "fallen" drop has an approx. volume of 60 - 70 µl
- Close the receiver plate with a lid and centrifuge the plate at 250x RCF for 30 sec- 1 min. This will allow to get rid of trapped air bubbles and the microtissue to center at the bottom of the well
- Check transfer efficiency under the microscope
- Medium Replenishment: aspirate the medium by placing the tips right at the ledge of the GravityTRAP plate and remove the medium
- Refill wells of GravityTRAP plate with 50-80 µl medium

### 1.4. Media used

| **Ingredient** | **Wash buffer** | **2D cell culture medium** | **Re-aggregation medium** |
|---|---|---|---|
| Williams E Medium (GE Healthcare) | X | X | X |
| Insulin-Transferrin-Selenium (Gibco) | X | X | X |
| FBS (Fetal Calf *Serum)* (Lonza) | 20% | 10 % | 20% |
| HGF (Hepatocyte Growth Factor) (Peprotech) | | | 20 µg/ml |
| 2mM Glutamine, Penicillin/Streptomycin, Amphothericin, 0.1 µM Dexamethasone | X | X | X |

### Example 2: Measuring Mitochondrial Toxicity with the Seahorse XF Analyzer

### 2.1 Treatment of 3D microtissues

After generating 3D primary human hepatocytes microtissues (IPHH_02) of a diameter size of 370um, the microtissues have been incubated with varying concentration of compounds for 48h Thereby, the compounds were dissolved in 3D InSight™ Human Liver Maintenance Medium -TOX

### 2.2 Seahorse XF Analyzer measurement

### A. Seahorse assay medium preparation

| **Component** | **Stock conc.(mM)** | **Assay conc. (mM)** | **Volume (mL)** |
|---|---|---|---|
| **DMEM XF Base** | - | - | 50 |
| **Glucose** | 1000 | 10 | 0.5 |
| **Na-Pyruvate** | 100 | 1 | 0.5 |
| **Ultra Glutamine** | 200 | 2 | 0.5 |
| Adjust pH to 7.4 | | | |

### B. Coating of Seahorse Spheroid plates

- 20uL of Poly-D-Lysine (100ug/mL diluted in cell culture grade water) were added to each well of the Seahorse Spheroid plate (Incubation for 20min @ RT)
- After coating, the Seahorse Spheroid plate was washed with 200uL of sterile water and wash was removed (Washing step repeated once)
- Seahorse Spheroid plate has been air dried for a few minutes

### C. Loading of Seahorse Spheroid plate with microtissues

- Warm up the prepared Seahorse assay medium (see above) to 37°C and fill up the plate with 70uL
- Use the Seahorse Capture Screen Insert Tool to accurately position the microtissues in the plate
- Flush the pipette tips several times with FCS before use
- After FCS-coating soak up the microtissues from the GravityTRAP plate in a volume of 20uL
- Transfer the pipette tips into the holes of the Seahorse Capture Screen Insert tool
- Leave the tips in the tool for about 1min to ensure complete spheroid transfer
- The spheroids are transferred to the wells by gravitation, so they fall out of the tips automatically and you do not have to use the trigger for that
- After completed microtissue transfer fill up the wells with pre-warmed Seahorse assay medium to a final volume of 180uL
- Scan the plates in the Insphero Cell3iMager to localize microtissues on the plate before the Seahorse run
- Incubate microtissues for 1h @ 37°C w/o CO₂

### D. Seahorse XF Analyzer run

- While cell incubation fill up the ports of the cartridge according to the Seahorse manufacturer's protocol
- Setup groups and parameters for the measurement (basal: 7 cycles, oligomycin 2uM: 6 cycles, FCCP 0.5uM: 3 cycles, FCCP 1uM: 3 cycles, Rot/AA 0.5uM: 9 cycles)
- Start the measurement

### E. Cell viability assessment

- In parallel to the Seahorse XF Analyzer run, cell viability has been measured of different microtissues treated the same way as for the Seahorse experiment

### E. Analysis and prediction of safety for Amiodarone as an example

- By generating a Sigmoidal dose-response curve (variable slope) of all Respiratory Spare Capacity values of each Amiodarone concentration, we were able to obtain an IC_{50_MTSRR} of 25.3µM
- By knowing that C_{Max} of Amiodarone is 5.3µM and dividing the IC_{50_MTSRR} by C_{Max}, we are able to generate a Margin of safety (MOS) of 4.8
- As this MOS of Amiodarone (4.8) is below 30, we have predicted Amiodarone to be a toxic compound, which correlates with the DILI classification (Gustaffson *et al.* 2013)
- Furthermore, we can identify the Mitochondrial toxicity risk range by considering the following scheme:
   ∘ IC_{5O_MTSRR} ≥ IC_{5O}__{CV/CYT} means that the candidate compound has no specific mitochondrial toxicity
   ∘ IC_{5O_MTSRR} < IC_{5O_CV/CYT} and IC_{5O_MTSRR} ≥ 0.75 x IC_{5O_CV/CYT} means that the candidate compound has a low risk of mitochondrial toxicity
   ∘ IC_{5O_MTSRR} < 0.75 x IC_{5O_CV/CYT} means that the candidate compound has a high risk of mitochondrial toxicity
- Amiodarone's IC_{5O_MTSRR} is smaller than 0.75 x IC_{5O_CV/CYT} which classifies Amiodarone as a compound with a high Mitotoxic risk

### Figures

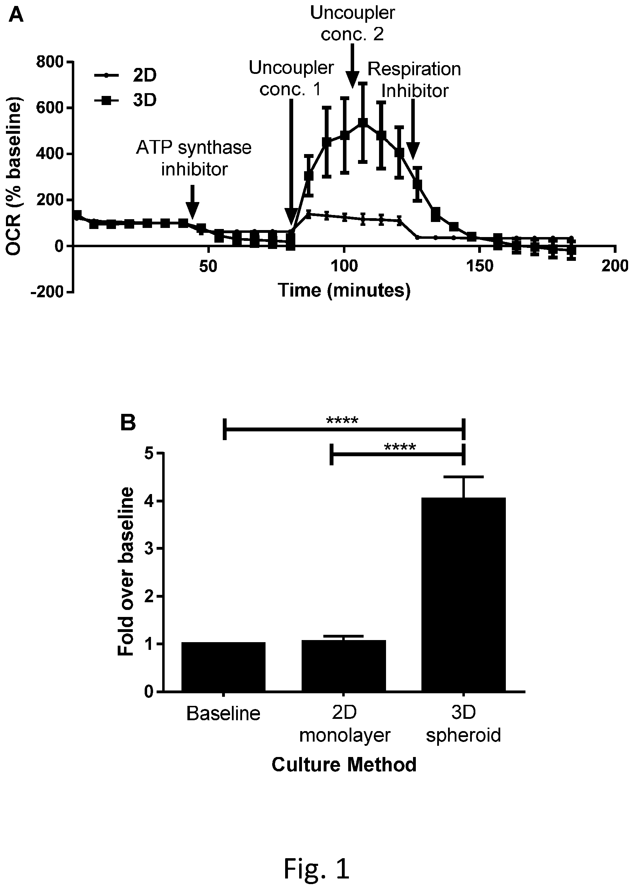

Fig. 1: Mitochondrial Bioenergetics 2D vs. 3D. A) OCR profile and B) Maximal Respiration. **** p < 0.0001 unpaired t-test, two-tailed, Means +/- s.e.m.

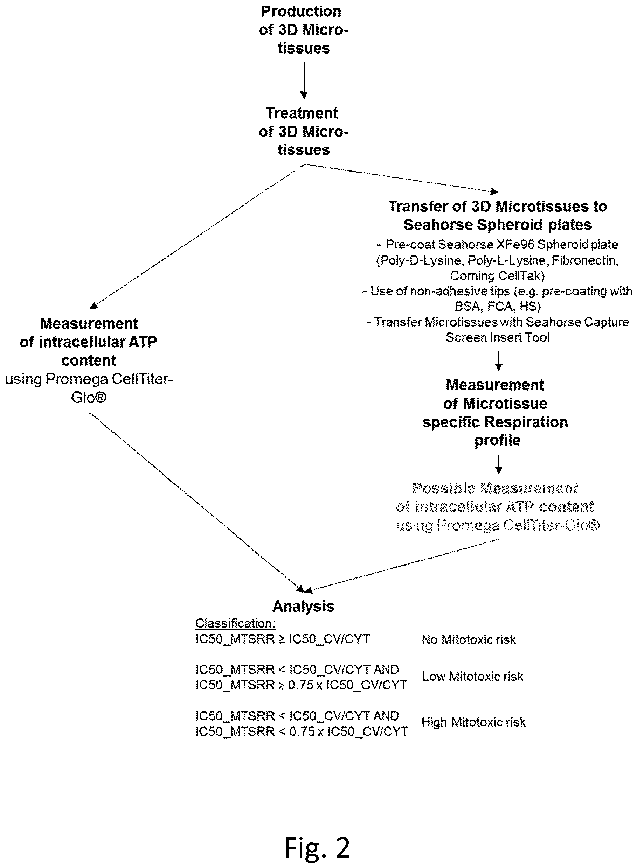

Fig. 2: Outline of a general workflow. The chronologic order of the different steps is just a preferred embodiment, meaning that the treatment of the 3D microtissues with the molecules to be screened can also take place synchronously with the subsequent assays on viability (ATP content) and respiration rate. Further and not depicted in the general workflow, the steps can be accompanied by a step of size determination, e.g., by digital image analysis. It is also important to mention that in some embodiments, the assays (viability, respiration rate and size) can be made with the same 3D microtissue, while in other embodiments different 3D microtissues are being used which have however been pretreated in identical fashion.

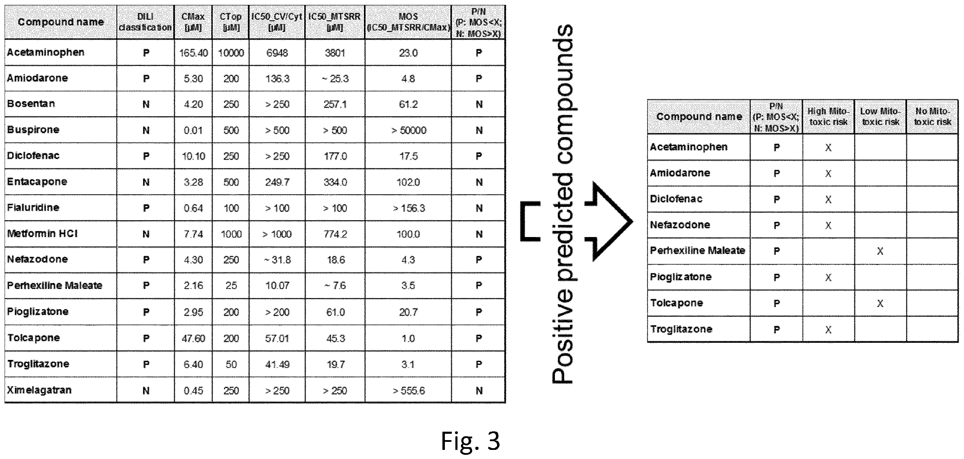

Fig. 3: Analysis example for 3D primary human hepatocyte microtissues exposed to different candidate compounds. DILI classification: Drug-induced liver injury according to Gustafsson et al, 2014 (N = negative, P = positive). CMax = concentration of candidate compound used in Gustafsson et al, 2014; CTop = concentration of candidate compound used herein; IC50_{CV/Cyt} determined by the CellTiter-Glo Luminescent Cell Viability Assay (Promega, Madison, US) as disclosed herein, IC50_{MTSRR} determined as inhibition on spare respiratory capacity (SPARE), as disclosed herein; MOS: Margin of safety; High Mito-toxic risk: IC_{5O_MTSRR} < 0.75 x IC_{5O_CV/CYT}; Low Mito-toxic risk: IC_{5O_MTSRR} < IC_{5O_CV/CYT} and IC_{5O_MTSRR} ≥ 0.75 x IC_{5O_CV/CYT}; No Mito-toxic risk: IC_{5O_MTSRR} ≥ IC_{5O_CV/CYT}

Asteriks(*) show candidate drugs with respect to which the instant assay method delivers a different and more precise result with respect to mitochondrial toxicity than the general DILI classification according to Gustafsson et al., 2014. Fialuridine, which was categorized as liver toxic by Gustafsson et al. turned out to not have specific mitochondrial toxicity, while Metformin HCl, which was categorized as not liver toxic by Gustafsson et al., turned out to have a low mitochondrial toxicity.

### References

Tsiper MV et al. (2012); PloS one vol. 7 (10) p. e45226
Gustafsson et al, (2013); Tox Sciences 137(1), 189-211

## Claims

1. A method for assessing the mitochondrial toxicity of a candidate compound, the method comprising
a) exposing one or more 3-dimensional cell culture or tissue to one or more candidate compounds, and
b) measuring, in at least one 3-dimensional cell culture or tissue, the effect of such exposure on the specific respiration rate of the 3-dimensional cell culture or tissue (microtissue-specific respiration rate, MTSRR),
c) measuring, in the same or at least one other 3-dimensional cell culture or tissue, the effect of such exposure on cell viability and/or the cytotoxic effect (CV-CYT) in one or more cells of at least one 3-dimensional cell culture or tissue, and
d) comparing the effects of steps b) and c) to provide an estimate on the mitochondrial toxicity of the one or more candidate compounds,
wherein the mitochondrial toxicity is determined on the basis of the quantitative relationship between
I) the IC₅₀ with respect to the specific respiration rate of the 3-dimensional cell culture or tissue (IC_{5O-MTSRR})
II) the IC₅₀ with respect to the cell viability/cytotoxicity (IC_{5O-CV/CYT}),
wherein
(i) IC_{5O_MTSRR} ≥ IC_{5O_CV/CYT} means that the candidate compound has no specific mitochondrial toxicity
(ii) IC_{5O-MTSRR} < IC_{5O_CV/CYT} and IC_{5O-MTSRR} ≥ 0.75 x IC_{5O_CV/CYT} means that the candidate compound has a low risk of mitochondrial toxicity
(iii) IC_{5O-MTSRR} < 0.75 x IC_{5O_CV/CYT} means that the candidate compound has a high risk of mitochondrial toxicity.

2. The method of claim 1, wherein the 3-dimensional cell culture or tissue
• is a spheroidal cell culture.

3. The method of claim 1 or 2, wherein the 3-dimensional cell culture or tissue comprises
• primary cells,
• hepatocytes or
• immortalized, malignant, cancerous and/or neoplastic cells.

4. The method according to any of claims 1 - 3, further comprising a step of determining, in the same or at least one other 3-dimensional cell culture or tissue, the effect of the exposure on the size thereof.

5. The method to claim 4, wherein determining the size refers to at least one parameter of:
• diameter
• perimeter
• volume
• area of an optical cross section.

6. The method according to any of claims 2 - 5, wherein the 3-dimensional cell culture or tissue further comprises at least one cell type of:
• Immune cells
• Stroma cells
• Fibroblasts
• Cancer stem cells
• Immortalized, malignant, cancerous and/or neoplastic cells that are known to be resistant or sensitive to a given anti-cancer agent.

7. The method according to any of the preceding claims, wherein the specific respiration rate of the 3-dimensional cell culture or tissue is determined by simultaneous measurement of the oxygen consumption rate (OCR) and the extracellular acidification rate (ECAR), of one or more isolated 3D microtissues.

8. The method according to claim 7, wherein in determining the microtissue-specific respiration rate (MTSRR), the spare respiratory capacity ("SPARE") is used.

9. The method according to claims 7 or 8, wherein in determining the microtissue-specific respiration rate (MTSRR), the basal respiration rate ("BASAL") and/or the maximal respiration rate ("MAX") are also used.

10. The method according to any of the preceding claims, wherein the measurement of cell viability/cytotoxic effect determines the residual viability of one or more cells.

11. The method according to any of the preceding claims, wherein exposure step a) is carried prior to subsequent measurement steps b) and/or c).

12. The method according to any of the preceding claims, wherein exposure step a) is carried out in one or more first vessels, while the subsequent measurement steps b) and/or c) are carried out in one or more second and/or third vessels.

13. The method according to claim 12, comprising after exposure step a) transferring the microtissues, by means of a suitable dispensing device, from the one or more first vessels to the one or more second and/or third vessels, to carry out measurement steps b) and/or c).

## Patentansprüche

1. Verfahren zur Beurteilung der mitochondrialen Toxizität einer Kandidatenverbindung, wobei das Verfahren aufweist
a) eine oder mehrere dreidimensionale Zellkulturen oder Gewebe einer oder mehreren Kandidatenverbindungen aussetzen und
b) in mindestens einer dreidimensionale Zellkultur oder einem Gewebe die Wirkung eines solchen Aussetzens auf die spezifische Respirationsrate der dreidimensionalen Zellkultur oder des Gewebes (mikrogewebespezifische Respirationsrate, MTSRR) messen,
c) in der gleichen oder wenigstens einer anderen dreidimensionalen Zellkultur oder einem anderen Gewebe die Wirkung eines solchen Aussetzens auf die Zellviabilität und/oder die zytotoxische Wirkung (CV-CYT) in einer oder mehreren Zellen mindestens einer dreidimensionalen Zellkultur oder eines Gewebes messen und
d) die Auswirkungen der Schritte b) und c) vergleichen, um eine Abschätzung der mitochondrialen Toxizität der einen oder mehreren Kandidatenverbindungen bereit zu stellen;
wobei die mitochondriale Toxizität auf der Grundlage der quantitativen Beziehung zwischen
I) der IC₅₀ in Bezug auf die spezifische Respirationsrate der dreidimensionalen Zellkultur oder des Gewebes (IC_{5O-MTSRR})
II) der IC₅₀ in Bezug auf die Zellviabilität/Zytotoxizität (IC₅₀-_{CV/CYT}),
bestimmt wird, wobei:
i) IC_{50-MTSRR} ≥ IC_{50-CV/CYT} bedeutet, dass die Kandidatenverbindung keine spezifische mitochondriale Toxizität hat
ii) IC_{50-MTSRR} < IC_{50-CV/CYT} und IC_{50-MTSRR} ≥ 0.75 x IC_{50-CV/CYT} bedeutet, dass die Kandidatenverbindung ein geringes Risiko für mitochondriale Toxizität hat
iii) IC_{50-MTSRR} < 0,75 x IC_{50-CV/CYT} bedeutet, dass die Kandidatenverbindung ein hohes Risiko für mitochondriale Toxizität hat.

2. Verfahren nach Anspruch 1, wobei die dreidimensionale Zellkultur oder das Gewebe
• eine sphäroidale Zellkultur ist.

3. Verfahren nach Anspruch 1 oder 2, wobei die dreidimensionale Zellkultur oder das Gewebe
• Primärzellen
• Hepatozyten oder
• immortalisierte, bösartige, cancerogene und/oder neoplastische Zellen aufweist.

4. Verfahren nach einem der Ansprüche 1 - 3, weiterhin einen Schritt aufweisend, in der gleichen oder wenigstens einer anderen dreidimensionalen Zellkultur oder einem anderen Gewebe die Auswirkung des Aussetzens auf deren Größe zu bestimmen.

5. Verfahren nach Anspruch 4, wobei die Größe bestimmen sich auf mindestens einen Parameter bezieht von:
• Durchmesser
• Umfang
• Volumen
• Fläche eines optischen Querschnitts.

6. Verfahren nach einem der Ansprüche 2 - 5, wobei die dreidimensionale Zellkultur oder das Gewebe weiterhin mindestens einen Zelltyp aufweist von:
• Immunzellen
• Stroma-Zellen
• Fibroblasten
• Krebsstammzellen
• immortalisierte, bösartige, cancerogene und/oder neoplastische Zellen, von denen bekannt ist, dass sie resistent oder empfindlich gegen ein bestimmtes Krebsmittel sind.

7. Verfahren nach einem der vorhergehenden Ansprüche, wobei die spezifische Respirationsrate der dreidimensionalen Zellkultur oder des Gewebes durch gleichzeitige Messung der Sauerstoffverbrauchsrate (OCR) und der extrazellulären Acidifizierungsrate (ECAR) von einem oder mehreren isolierten 3D-Mikrogeweben bestimmt wird.

8. Verfahren nach Anspruch 7, wobei beim Bestimmen der mikrogewebespezifischen Respirationsrate (MTSRR) die freie Respirationskapazität ("SPARE") verwendet wird.

9. Verfahren nach den Ansprüchen 7 oder 8, wobei beim Bestimmen der mikrogewebespezifischen Respirationsrate (MTSRR), ebenfalls die basale Respirationsrate ("BASAL") und/oder die maximale Respirationsrate ("MAX") verwendet werden.

10. Verfahren nach einem der vorhergehenden Ansprüche, wobei die Messung der Zellviabilität /zytotoxischen Wirkung die Restviabilität einer oder mehrerer Zellen bestimmt.

11. Verfahren nach einem der vorhergehenden Ansprüche, wobei der Schritt des Aussetzens a) vor den nachfolgenden Messschritten b) und/oder c) durchgeführt wird.

12. Verfahren nach einem der vorhergehenden Ansprüche, wobei der Schritt des Aussetzens a) in einem oder mehreren ersten Behältnissen durchgeführt wird, während die nachfolgenden Messschritte b) und/oder c) in einem oder mehreren zweiten und/oder dritten Behältnissen durchgeführt werden.

13. Verfahren nach Anspruch 12, welches nach dem der Schritt des Aussetzens a) aufweist, die Mikrogewebe von dem einen oder den mehreren ersten Behältnissen durch eine geeignete Dosiervorrichtung auf das eine oder die mehreren zweiten und/oder dritten Behältnisse zu übertragen, um die Messschritte b) und/oder c) durchzuführen.

## Revendications

1. Procédé pour évaluer la toxicité mitochondriale d'un composé candidat, le procédé comprenant
a) l'exposition d'une ou plusieurs cultures ou tissus cellulaires à 3 dimensions à un ou plusieurs composés candidats, et
b) la mesure, dans au moins une culture ou tissu cellulaire à 3 dimensions, de l'effet d'une telle exposition sur le rythme respiratoire spécifique de la culture ou tissu cellulaire à 3 dimensions (rythme respiratoire spécifique à un microtissu, MTSRR),
c) la mesure, dans la même ou au moins une autre culture ou tissu cellulaire à 3 dimensions, de l'effet d'une telle exposition sur la viabilité cellulaire et/ou de l'effet cytotoxique (CV-CYT) dans une ou plusieurs cellules d'au moins une culture ou tissu cellulaire à 3 dimensions, et
d) la comparaison des effets des étapes b) et c) pour fournir une évaluation de la toxicité mitochondriale du ou des plusieurs composés candidats,
dans lequel la toxicité mitochondriale est déterminée sur la base de la relation quantitative entre
I) l'IC₅₀ en ce qui concerne le rythme respiratoire spécifique de la culture ou tissu cellulaire à 3 dimensions (IC50-MTSRR)
II) l'IC₅₀ en ce qui concerne la viabilité/cytotoxicité cellulaire (IC_{50-CV/CYT}),
dans lequel
(i) IC_{50-MTSRR} ≥ IC_{50-CV/CYT} signifie que le composé candidat n'a aucune toxicité mitochondriale spécifique
(ii) IC_{50-MTSRR} < IC_{50-CV/CYT} et IC_{50-MTSRR} ≥ 0,75 × IC_{50-CV/CYT} signifie que le composé candidat a un faible risque de toxicité mitochondriale
(iii) IC_{50-MTSRR} < 0,75 x IC_{50-CV/CYT} signifie que le composé candidat a un risque élevé de toxicité mitochondriale.

2. Procédé selon la revendication 1, dans lequel la culture ou tissu cellulaire à 3 dimensions
• est une culture cellulaire sphéroïdale.

3. Procédé selon la revendication 1 ou 2, dans lequel la culture ou tissu cellulaire à 3 dimensions comprend
• des cellules primaires,
• des cellules hépatiques ou
• des cellules immortalisées, malignes, cancéreuses et/ou néoplasiques.

4. Procédé selon l'une quelconque des revendications 1 à 3, comprenant en outre une étape de détermination, dans la même ou au moins une autre culture ou tissu cellulaire à 3 dimensions, de l'effet de l'exposition sur la taille de celle-ci.

5. Procédé selon la revendication 4, dans lequel la détermination de la taille se réfère à au moins un paramètre parmi :
• le diamètre
• le périmètre
• le volume
• la surface d'une section transversale optique.

6. Procédé selon l'une quelconque des revendications 2 à 5, dans lequel la culture ou tissu cellulaire à 3 dimensions comprend en outre au moins un type particulier de cellule parmi :
• des cellules immunitaires
• des cellules du stroma
• des fibroblastes
• des cellules-souches cancéreuses
• des cellules immortalisées, malignes, cancéreuses et/ou néoplasiques que l'on connaît pour être résistantes ou sensibles à un agent anticancéreux donné.

7. Procédé selon l'une quelconque des revendications précédentes, dans lequel le rythme respiratoire spécifique de la culture ou tissu cellulaire à 3 dimensions est déterminé par une mesure simultanée du taux de consommation d'oxygène (OCR) et du taux d'acidification extra-cellulaire (ECAR), d'un ou plusieurs microtissus 3D isolés.

8. Procédé selon la revendication 7, dans lequel lors de la détermination du rythme respiratoire spécifique à un microtissu (MTSRR), la capacité respiratoire de réserve (« SPARE ») est utilisée.

9. Procédé selon les revendications 7 ou 8, dans lequel lors de la détermination du rythme respiratoire spécifique à un microtissu (MTSRR), le rythme respiratoire basique (« BASAL ») et/ou le rythme respiratoire maximal (« MAX ») sont également utilisés.

10. Procédé selon l'une quelconque des revendications précédentes, dans lequel la mesure de la viabilité/effet cytotoxique cellulaire détermine la viabilité résiduelle d'une ou plusieurs cellules.

11. Procédé selon l'une quelconque des revendications précédentes, dans lequel l'étape d'exposition a) est effectuée avant les étapes de mesure suivantes b) et/ou c) .

12. Procédé selon l'une quelconque des revendications précédentes, dans lequel l'étape d'exposition a) est effectuée dans un ou plusieurs premiers récipients, tandis que les étapes de mesure suivantes b) et/ou c) sont effectuées dans un ou plusieurs deuxièmes et/ou troisièmes récipients.

13. Procédé selon la revendication 12, comprenant après l'étape d'exposition a) le transfert des microtissus, au moyen d'un dispositif de distribution approprié, depuis le ou les plusieurs premiers récipients vers le ou les plusieurs deuxièmes et/ou troisièmes récipients, pour effectuer les étapes de mesure b) et/ou c) .
